# EUROPEAN PATENT SPECIFICATION

(11) **EP 0 823 887 B1**
(45) Date of publication and mention of the grant of the patent: **20.03.2002**
(21) Application number: 96919655.9
(22) Date of filing: 02.05.1996
(51) Int. Cl.: C05F 17/00, C05F 7/00, C02F 3/04, C02F 3/28, C12M 1/107

(54) **METHOD AND DEVICE FOR A COMBINED TREATMENT OF DOMESTIC SEWAGE AND DOMESTIC BIODEGRADABLE GARBAGE**
VERFAHREN UND VORRICHTUNG ZUR KOMBIENIERTEN BEHANDLUNG VON KOMMUNALEM ABWASSER UND BIOLOGISCH ABBAUBAREM HAUSMÜLL
PROCEDE ET DISPOSITIF DE TRAITEMENT COMBINE DES EAUX D'EGOUT MENAGERES ET DES DECHETS MENAGERS BIODEGRADABLES

(30) Priority: 02.05.1995 LK 1080195; 14.06.1995 LK 1083095; 27.07.1995 EP 95111851
(43) Date of publication of application: 18.02.1998
(73) Proprietor: Braun, Ulrich, 66117 Saarbrücken (DE)
(72) Inventor: Braun, Ulrich, 66117 Saarbrücken (DE)
(74) Representative: VOSSIUS & PARTNER
(86) International application number: EP9601831
(87) International publication number: WO9634841

(56) References cited:
- WO-A-93/14047
- CH-A- 241 188
- DE-A- 3 015 239
- DE-A- 3 602 860
- DE-C- 3 821 028
- FR-A- 874 543
- FR-A- 2 541 669
- FR-A- 2 708 588
- US-A- 3 494 463
- US-A- 4 318 993

## Description

This invention regards to a method and device to treat the entire organic refuse of preferably domestic settlements, both liquid (sewage, primary-, secondary- and tertiary sewage sludge) and solid biodegradable garbage. The name of the invention is T&U BioConverter.

There are several large-scale sewage treatment methods and devices on the market. They have high running costs in common. The sewage sludge fermenting towers are running with a dry substance content between 3% and 5%. Financially, the utilisation of the biogas from the sewage is feasible from 50,000 PE (= Population Equivalents) upwards. Small scaled sewage plants (known as Schreiber Klaerwerk, Schachtelbecken, VEDEWA-Rundbecken, Essener Becken, Gegenstrom Rundbecken, Biosedimat, Gyromat S, Oxigest-Klaeranlage, Total-Klaeranlage, etc.) are rarely utilising the biogas. The anaerobic fermentation of the sewage sludge has its main purpose in sludge conditioning.
Thus the disadvantage of the existing large-scale and small-scale methods and devices of sewage treatment plants is the low yield of biogas and high construction and running costs.

General biodegradable garbage treatment methods and devices are aerobic composting, anaerobic fermentation and aerobic composting, and incineration or pyrolysis. Aerobic composting methods and devices are common in a large and small scale. Incineration, pyrolysis and anaerobic fermentation are common only in large-scale methods and devices. The anaerobic fermentation of biodegradable garbage is done in a liquid form with dry substance content between 8% and 12%, and in a wet form with a dry substance content between 25% and 45%. Both anaerobic fermentation methods are producing excess process water which is polluted and requires treatment.
The disadvantage of incinerating or pyrolysing unsorted garbage is the loss of fertilising substances, the toxic emissions and ashes, and the high energy demand to evaporate the water content which originally is 65% - 75% of the biodegradable garbage.
The disadvantage of large-scale fermentation and composting methods and devices of biodegradable garbage treatment plants are:
a. the long ways of the biodegradable garbage, leading to a loss of volatile solids due to microbiological activity and thus a loss of biogas. The state of art yield of biogas is normally around 110 Nm³ per metric ton of biodegradable garbage.
b. the additional demand of a waste water treatment plant on site.

It has been proposed, to build a sewage plant and a garbage treatment plant separated, but on the same site (Patent DE-24 60 480).
The disadvantage to treat sewage and biodegradable garbage in separated plants are:
c. space demand in case of two separated plants is higher,
d. costs for technical equipment for two plants are higher, and
e. the volume and costs of two fermentation towers, one with a dry substance content of approx. 5%, one with approx. 10%, are high.

Patent DE-44 03 589 proposes to feed the shredded biodegradable garbage into already existing fermentation towers of sewage plants.
The disadvantage is the lack of the hydrolytic step as to improve the efficiency of anaerobic catabolism, besides the heavy development of floating sludge in the fermentation towers (disadvantageous volume - upper sludge surface ratio).

There are several patents referring to combined treatment of sewage sludge and biodegradable garbage. Patents DE-24 31 120, DE-29 09 515, DE-30 01 508, DE-30 46 646, DE-32 00 915 and DE-39 16 866 are describing methods, which are characterised in that the separated sludge together with the garbage undergoes a composting device without production of biogas. Patent DE-38 38 894 is proposing a mobile two chamber device for drying and aerobic thermal treatment. The disadvantages are here not to utilise the volatile solids of the organic matter as biogas.

There are also several patents referring to combined waste water and garbage treatment. Patents DE-OS 22 60 616, DE-28 34 718 and patent DE-25 58 703 are proposing methods to separate the sludge and incinerate it combined with the garbage for the production of carbon, or activated carbon, which are then used again in the water treatment device as a filter, or being sold as activated carbon. The disadvantages here are the high costs of the method. The evaporation energy of the sludge and garbage mixture is very high.
Patent DE-34 28 716 is proposing an incineration of the sludge and garbage. Again the disadvantage here is the high costs factor.
Patent DE-38 38 894 is proposing a mobile disposal method for sewage sludge combined with the garbage. The disadvantage here is the restriction of the size of the plant.
Patents DE-36 27 253 and DE-38 43 789 are proposing a method for combined treatment of preferably liquid manure and biodegradable garbage, which is characterised by a sedimentation step between the hydrolysis step and the methane fermentation step. The disadvantage here lies in the big volume necessary for the hydrolysis device, and the bad sedimentation characteristics of partly fermented sludge.
Patent DE-42 02 326 proposes a method which is characterised in that the liquid from the solid phase is separated. The solids are then treated aerobically and the liquid phase undergoes first an anaerobic phase and is finally treated aerobically. The disadvantage here is again the lack of biogas production.
Patents SHO 60-29 319 and HEI 1-60 313 (Japan) propose a method and device for decentralised and compact treatment of biodegradable garbage and sewage. Both patents disclose aerobic sludge and garbage treatment. As sewage treatment, a multi staged activated sludge process is proposed. The disadvantage here are the costs and the lack of biogas production.
Patent DE-42 02 327 A1 discloses a method and device for decentralised and compact treatment of sewage and biodegradable garbage which is characterised in single stage anaerobic fermentation of primary sewage sludge and biodegradable garbage with internal stirrer. To achieve an increase of the solubilization of eg. fibrous organic matter, a treatment with fluoride is proposed. To increase the anaerobic catabolic process, an addition of a stimulating agens is proposed. As a flotation save-all, a retaining mesh is suggested. As sewage treatment a multi-staged activated sludge process is proposed. The disadvantage here are the production of FCC, the lack of a hydrolytic step, the costly devices and running costs, and the tendency of clogging of the retaining mesh in the anaerobic reactor.

It is the object of the present invention to overcome the above disadvantages and to provide a method and device for the means for decentralised and combined treatment of domestic sewage and anaerobic treatment of domestic biodegradable garbage In one device by taking maximised usage of the solar thermal energy and the gassing pressure energy of the biogas. Furthermore, the invention of this method and device preferably keeps the space requirement, the construction and running costs of the treatment plant as low, and at the same time the output of useful products as high as possible as to improve the economical balance.

This object is fulfilled with the means of the claims.

As commonly known, the oxygen solubility in water is decreasing, and the catabolic microbiological efficiency is increasing, within the physiologicl ranges, with increasing temperatures . Additionally, the temperature differences in tropical areas are much less than in moderate climates. In Europe, for example, the air stream in trickling filters is often downwards during the day, and upwards during the night due to the often found difference of 10°C and more between the waste water and air. In tropical areas, the temperature difference between waste water and air, as well as between day and night is much less. A result thereof in case of e.g. modem trickling filters with a high inner surface - volume ratio (over 180 m²/m³) in tropical areas is, that often large areas within the trickling filter are running under anaerobic conditions. This leads to a decreased efficiency of the water treatment facility, to offensive odour emission and to bad sedimentation properties of the secondary sedimentation step. Generally, the antagonists oxygen solvability and microbiological activity in hot areas demand intense aeration in order to take advantage of the high microbiological activity.
Also in moderate climatic conditions, the subject matter of this invention achieves better resistance against cold temperatures during the winter due to the heat input and the restricted and controllable heat losses of the device according to the invention.
The chimney effect is caused by heat accumulation in a partly closed space, e.g. a pipe, which is resulting in an updraft, caused by the lower specific weight of the warmed up liquid or gasiform substance.
A welcomed side effect of the chimney effect preferably used by the present invention is the utilisation of the primary waste water treatment device (in case of being a trickling filter, RBC process, etc.) as a biological air filter to minimise the offensive odours caused by the sedimentation stages and the drainage and/or drying device.
The air stream of the chimney effect can additionally be used for supportive transportation of the floating sludge blankets of the two sedimentation stages to the blow off pipe.
Well known is the chimney effect of trickling filters. The excess heat gained by aerobic catabolism creates an updraft in the trickling filter transporting oxygen into the filter. Purposeful strengthened usage of the chimney effect for water treatment can only be found in water desalination methods.

Hotels in tropical countries are producing a special garbage, which has similar characteristics in most cases of other tourist areas being investigated. The garbage has a high content of organic matter (approx. 85% of fresh weight). Due to the left overs of e.g. buffets and restaurants the content of volatile solids (easy biodegradable material) is extraordinarily high. Additionally, the amount of fats and grease is due to the high excess production in the kitchen also extraordinarily high.
An other special characteristic is the high content of fresh garden and park foliage. Most parks and gardens in hotels are well-groomed and produce a high amount of fresh and green, but fibrous material.
A sufficient technical know-how of handling and running combined sewage and garbage treatment plants is normally not existing in tourist areas. Thus the device must be easy to handle and must be insusceptible against disturbances.

The common environmental consciousness is also poorly spread in most tourist destinations. Advantage of the present invention is the profitability of the recycling system. Thus the method and device should be reasonably priced, the running costs should be minimised and the output of useful products should be maximised.

To integrate the method and device in several decentralised units in cities even in industrialised countries, the device should require as little space as possible. The separated treatment of the organic compound of municipal garbage combined with waste water treatment in one decentralised device with minimised construction and running costs can achieve profitability, and is additionally improving the energetic and financial balance as well as the long-term capacity of existing garbage incinerators.

The fundamental idea of the invention is a method and device for the means for combined treatment of preferably domestic waste water and of preferably domestic biodegradable garbage in one space saving device as to decrease construction costs and to decrease running costs by utilising solar thermal and gassing pressure energy and at the same time increasing the output of reusable and/or sellable products (biogas, compost, water) as to make environmental protection measures financially attractive.
The device is a compact plant, preferably comprising mechanical (rake, flat sand trap, two sedimentation stages), primary (BOD₅ and COD removal, nitrification) and secondary (denitrification and removal of phosphorus) means for water cleansing and the means for methane fermentation of primary sewage sludge, secondary bacterial sludge and tertiary excess sludge (optionally) combined with the shredded biodegradable domestic garbage and the drainage and/or drying of the fermented organic matter. The method and device for the means of sewage purification can be extended up to the production of drinking water.
In order to minimise the space demand of the invention and as to improve the biological efficiency of the means for primary waste water cleansing (BOD₅ and COD removal, and nitrification) and thus the costs for its construction, as well as to minimise the space demand of the drainage and/or drying device, the device according to the invention is preferably built as a tower; thus utilising and strengthening the chimney effect, caused by heat accumulation under the transparent cover of the drainage and/or drying device as well as the microbiological excess heat caused by aerobic catabolism.

Preferably, the method and device for the means for combined treatment of domestic sewage and anaerobic treatment of domestic biodegradable garbage in one device is equipped with a continuously anaerobic reactor for domestic biodegradable garbage and primary and secondary sewage sludge fermentation according to the functional principle of fixed dome plants, which are running very successfully even in rural areas of developing countries. The advantages of fixed dome plants are the reasonable construction costs, the process stability and easy control of the plant with the timing of the biogas release. A fixed dome plant comprises a closed anaerobic reactor, in which the developing biogas is accumulating at the top of the reactor, and tank for pressure equalisation of the closed anaerobic reactor, in which the sludge volume, displaced by the biogas accumulation in the anaerobic reactor, flows in. The displaced sludge volume flows back into the anaerobic reactor by releasing the biogas. The turbulence of the back and forth flow of the organic solution together with the inflow turbulence of the fresh organic matter is continuously stirring the sludge in the anaerobic reactor. To avoid sedimentation, both anaerobic reactor and pressure equalisation tank can have funnel shaped bottoms, with the blow off pipe end at the funnel top. A stirrer for the continuously anaerobic reactor can be added.

Furthermore, the device according to the invention is preferably equipped in the continuously anaerobic reactor with a floating sludge blanket blow off chamber and -pipe, and a floating sludge blanket transportation system, with which the sludge blanket is transported towards the blow off chamber on transportation bars, which are attached to the inner walls of the reactor, by utilising the changing sludge levels in the reactor caused by the produced and released biogas. This floating sludge transportation and removal device is useful due to the fibrous organic matter input.

Furthermore, the device according to the invention is preferably equipped with an inlet and mixing tank for the continuously anaerobic reactor, which simultaneously functions as a hydrolysis reactor.

Additionally, the device according to the invention is preferably executed in a way that no or a few electrical devices (e.g.. pump for a trickling filter, electric motor for a RBC tank, etc.) are necessary for the entire material flow within the device according to the invention. In case of waste water treatment with a non-technical and natural device (soil filter, fish tank, etc.) no electrical machine is necessary, and the energy consumption of the device according to the invention is zero.

The objects, advantages and characteristics of the invention will be shown and exemplified by means of the drawings.
Fig. 1: Embedding of the device according to the invention in an exemplary treatment system
Fig. 2: Material flow in a device according to the invention
Fig. 3: Exemplary layout plan of a device according to the invention
Fig. 4: Exemplary construction plan of a device according to the invention
Fig. 5: Exemplary sedimentation device within a device according to the invention
Fig. 6: Exemplary sludge blow off device from sedimentation stage 1 and 2
Fig. 7: Exemplary continuously anaerobic fixed dome reactor
Fig. 8: Floating sludge blanket removal
Fig. 9: Floating sludge blanket removal
Fig. 10: Floating sludge blanket removal
Fig. 11: Floating sludge blanket removal
Fig. 12: Floating sludge blanket removal
Fig. 13: Cross section through the continuously anaerobic reactor
Fig. 14: Floating sludge blanket transport: Situation before gas release
Fig. 15: Floating sludge blanket transport: Gas release
Fig. 16: Floating sludge blanket transport: Gas released
Fig. 17: Floating sludge blanket transport: Situation before floating sludge blanket removal
Fig. 18: Floating sludge blanket transport: Floating sludge blanket removal
Fig. 19: Floating sludge blanket transport: Floating sludge blanket removed
Fig. 20: Air flow in an device according to the invention

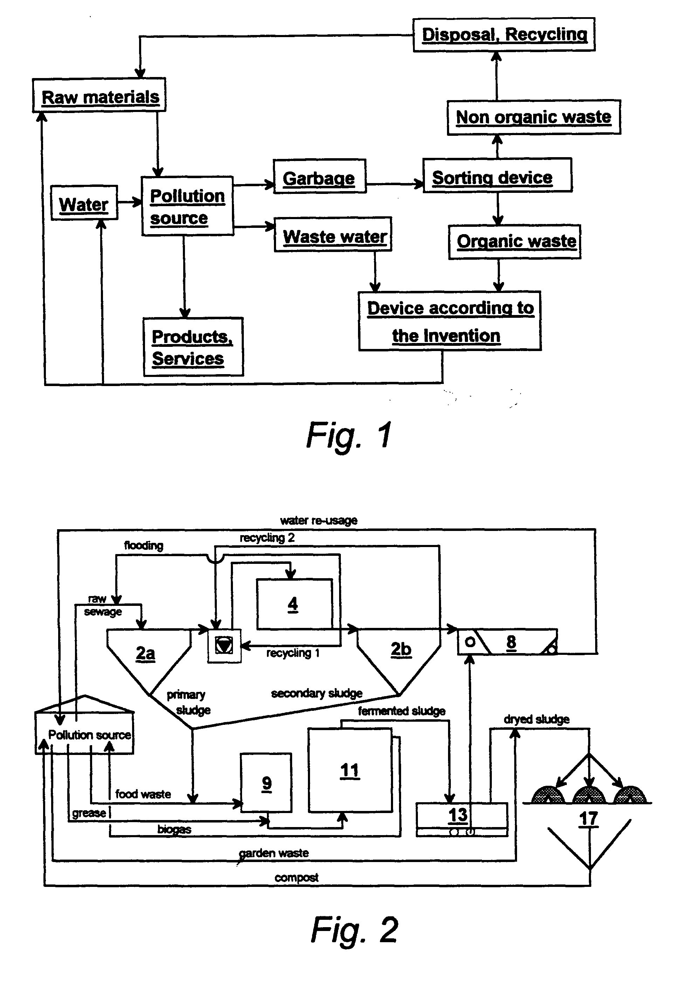

Fig. 1 shows the embedding of the device according to the invention in a entire recycling system. The device according to the invention is combined in one building, which is the subject of this patent application: The design of the device according to the invention can be modified and embedded in a any treatment and or recycling system. The entire treatment system shown in Fig. 1 is only exemplary of the invention.

Fig. 2 shows the material flow within the device according to the invention in connection with the pollution source. Raw sewage is entering the first sedimentation device 2a, is then treated in the primary treatment device 4 via the pump sump 3 and then flowing into the second sedimentation device 2b. There are three recycling possibilities. Recycling 1 gives the opportunity to treat the pre-treated secondary bacterial sludge containing effluent again in the primary treatment device 4. Recycling 2 provides the opportunity to treat the pre-treated secondary bacterial sludge free effluent (it has passed the secondary sedimentation device 2b) also again in the primary treatment device 4. The flooding arrow recycles the pre-treated effluent back into the first sedimentation device, thus preventing odour emission as well. The pre-treated and sludge free effluent flows then into the secondary treatment device 8. From here, the effluent has recycling quality, and is pumped back to the user after chlorinating.
The sludge gained by the first 2a and second 2b sedimentation device are combined with the food waste (and fresh foliage from the garden) entering the hydrolysis and/or mixing tank 9. After hydrolysis, the first step of catabolism, the organic solution flows into the methane fermenter 11. The fat and grease is directly fed into the methane fermenter, because the hydrolysis of fats and grease is the biochemical speed limiting step.
After fermenting with a composition specific retention time in the methane fermenter 11, the organic solution is overflowing into the drainage and/or drying device 13. In case of a drainage device 13, the drained water is flowing back to the secondary treatment device due to the high COD/BOD₅ ratio. The dried organic matter is then aerobically further biodegraded on composting heaps, from where it also can be fed back for reuse to the pollution source.

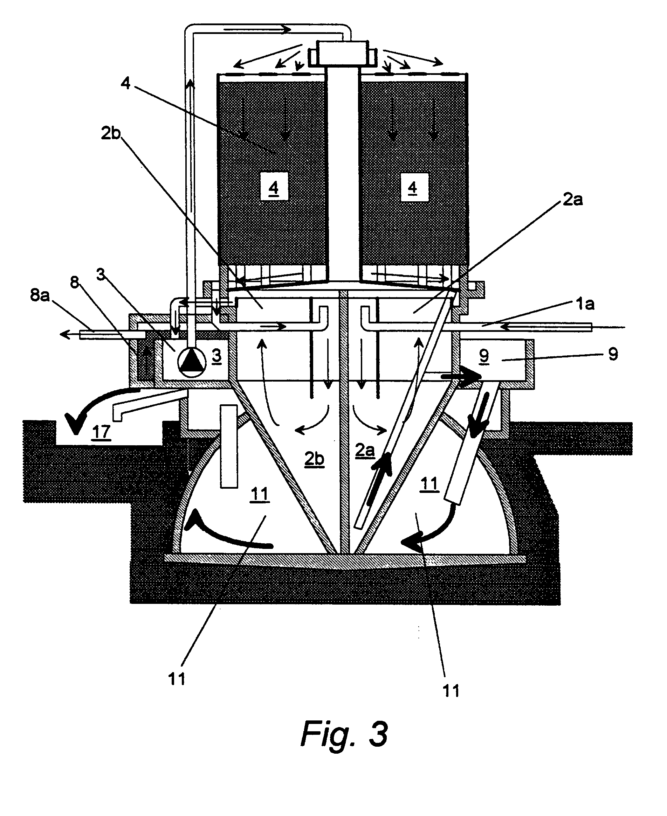

Fig. 3 shows an exemplary functional draft of the tower-like construction of the invention. The space demand of this draft is 0.05 m² per PE (= population equivalents). It is referred to Fig. 2, and the list of devices. The thin arrows are representing the water flow, the thick arrows the sludge and garbage flow. The raw sewage is entering via inlet pipe 1a the first sedimentation device 2a, the primary 4 and secondary 8 treated effluent is eluting via outlet pipe 8a for further treatment or direct recycling after chlorinating. The shredded organic waste combined with the sludge are flows, mediated by gravity from the hydrolysis device 9 through the methane fermenter 11 into the drainage and/or drying device 13. The primary 2a and secondary 2b sedimentation devices are here combined in one divided Dortmund Tank. The primary and secondary sludge are accumulating at the funnel top of the device. The methane fermenter 11 is build around the Dortmund Tank (2a and 2b), and the primary treatment device 4 is build on top of the Dortmund Tank (2a and 2b). All other devices ( Hydrolysis device 9, pump sump 3, secondary treatment device 8 and pressure equalisation device 12) are preferably build around this central tower.

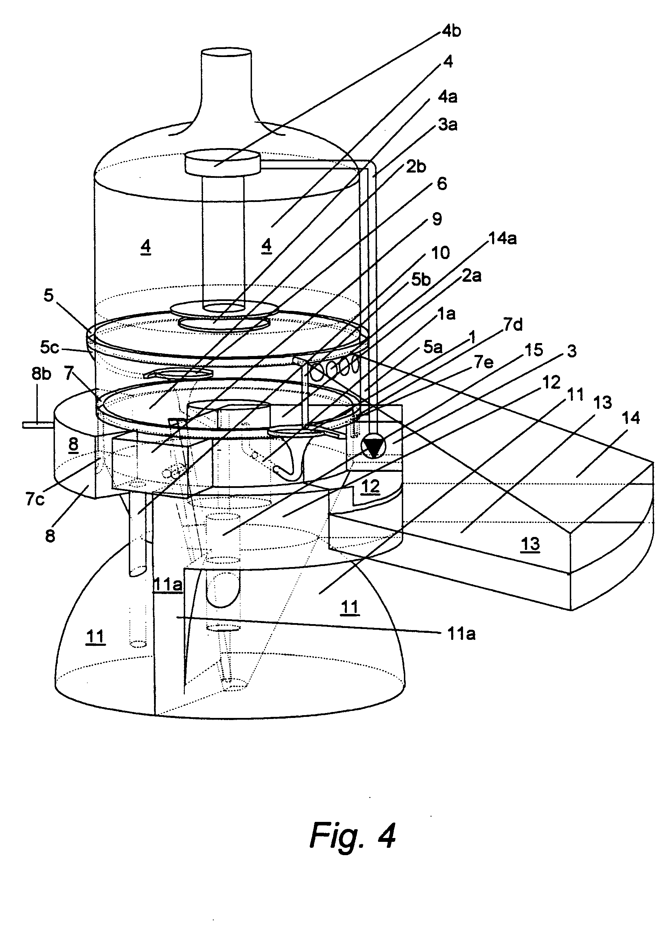

Fig. 4 shows the complete constructional plan of the exemplary draft of Fig. 3. It is referred to Fig. 2, Fig. 3 and the list of devices. There is provided a connection pipe 5bfor flooding the first sedimentation device 2a with fresh, oxygen enriched pre-treated effluent for odour minimisation. Pipe 5a can achieve a close loop between primary treatment device 4 and the pump sump 3. The single compounds of the invention will be explained in the following drawings.

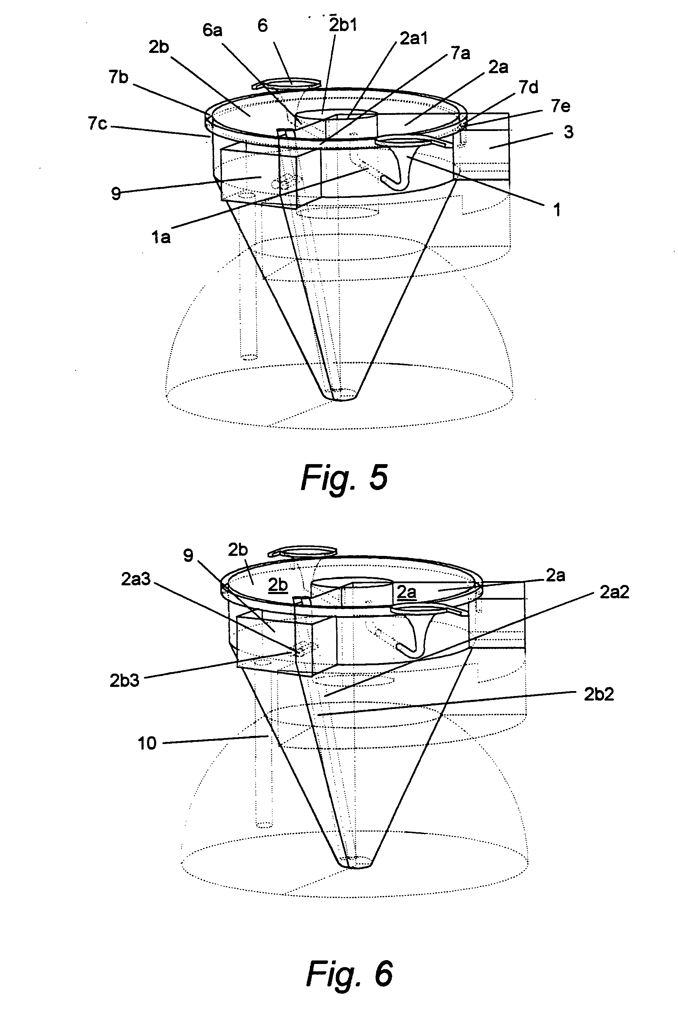

Fig. 5 shows the sedimentation unit with pump sump 3 and hydrolysis device 9. The raw sewage flows in through inlet building 1, connection pipe 1a into inlet cylinder 2a1. Here the effluent is forced to flow downwards, and then to flow upwards in sedimentation device 2a, and overflowing into the gutter 7a. The only outlet of gutter 7a is leading into the pump sump 3. After primary treatment 4 (see also Fig. 4), the pre-treated effluent flows in through inlet building 6, connection pipe 6a into inlet cylinder 2b1. Here also, the effluent is forced to flow downwards, then to flow upwards in sedimentation device 2b and then overflowing into gutter 7b. Gutter 7b has two outlets: Pipe 7c is feeding the secondary treatment device 8 with the pre-treated, sludge free effluent for further treatment. Pipe 7e can achieve a close loop (if pipe 7c is dosed) between pump sump 3, primary treatment 4 and secondary sedimentation 2b. Both pipes 7e and 5a make the entire plant extraordinary flexible, and easy adjustable to different effluent quantities. The dosed loop via pipe 7e is preferably used for flushing and cleansing of the primary treatment device 4. The outflowing pre-treated effluent quantities in case of pipe 7c and 7e being both open can be adjusted with a slider and different sashes. Independent from the position of the slider, 100% of the effluent quantity entering the system via inlet building 1 is flowing off through pipe 7c. With the pipe 7e the hydraulic load of the primary treatment device 4 and the secondary treatment device 2b can be changed at the same time, whilst the pipe 5a (see Fig. 4) can change the hydraulic load of the primary treatment device 4 without affecting the secondary treatment device 2b. The adjustments of the different recycling rates according to the inflowing waste water quantity can easily be automated with a water level sensor at the venturi channel, electric controllable valves and sliders and a computer.
In both sedimentation devices, the primary sludge in 2a and secondary sludge in 2b are accumulating at the funnel top.

Fig. 6 shows the sludge blow off devices of the sedimentation stages 2a and 2b. Both blow off devices are located besides the separation wall in the exemplary Dortmund Tank. Opening the valve of pipe 2a3 forces the primary sludge due to the water pressure of sedimentation device 2a to flow through pipe 2a2 into hydrolysis device 9. Opening the valve of pipe 2b3 forces the secondary sludge due to the water pressure of sedimentation device 2b to flow through pipe 2b2 into hydrolysis device 9.
Sewage sludge has a specific weight of 1.15 to 1.2, and sewage water a specific weight of approx.1.05. With a floater with a specific weight of 1.12, or other appropriate measurement devices, the sludge levels in both sedimentation devices 2a and 2b can be measured and the release can easily be automated with the same tools (see explanations of Fig. 5) as well.

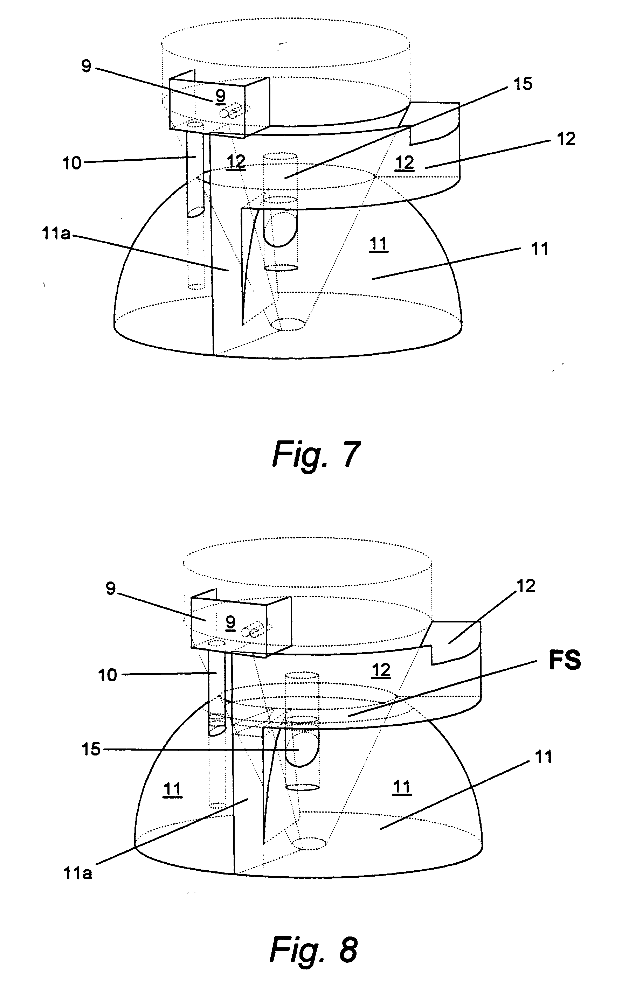

Fig. 7 shows the sludge and shredded biodegradable garbage treatment unit. As mentioned before (see Fig. 6), the sludges from sedimentation device 2a and 2b are flowing into the hydrolysis device 9. After adding the garbage, the dry substance content is approx. 15%. By releasing more sewage out of the sedimentation devices 2a and 2b, the dry substance content can be adjusted to around 10%. During hydrolysis the pH in the solution decreases from 7.5 down to 2.5. Due to the acid sensitivity of the methane bacteria in the methane fermenter, lime milk (Ca(OH)₂), or other lye can be added as to neutralise the pH. Before releasing the organic solution into the methane fermenter 11 after a composition depending retention time for complete hydrolysis and eventually pH adjustment, the fats and grease are added. After stirring, the organic solution is fed into the methane fermenter by opening the valve or slider of pipe 10. This pipe has preferably a diameter of at least 20 cm in order to achieve with a fast flow a high kinetic energy input into the methane fermenter 11 for intermixing the fermenting liquor. Additionally, the organic solution should be released after releasing the biogas from the methane fermenter 11 as to maximise the hydraulic drop between the hydrolysis device 9 and the sludge level in the pressure equalisation tank 12. The bottoms of the hydrolysis device 9, as well as the methane fermenter 11 and the pressure equalisation tank 12 can be designed in a funnel shape with the outlet pipes at the funnel top as to avoid sediment accumulation. The hydrolysis device can also be equipped with an active biomass retaining device as to improve the efficiency of the batch wise hydrolytic process. The entire anaerobic unit shown can also be designed for a continuously material flow. This device can also easily be automated.

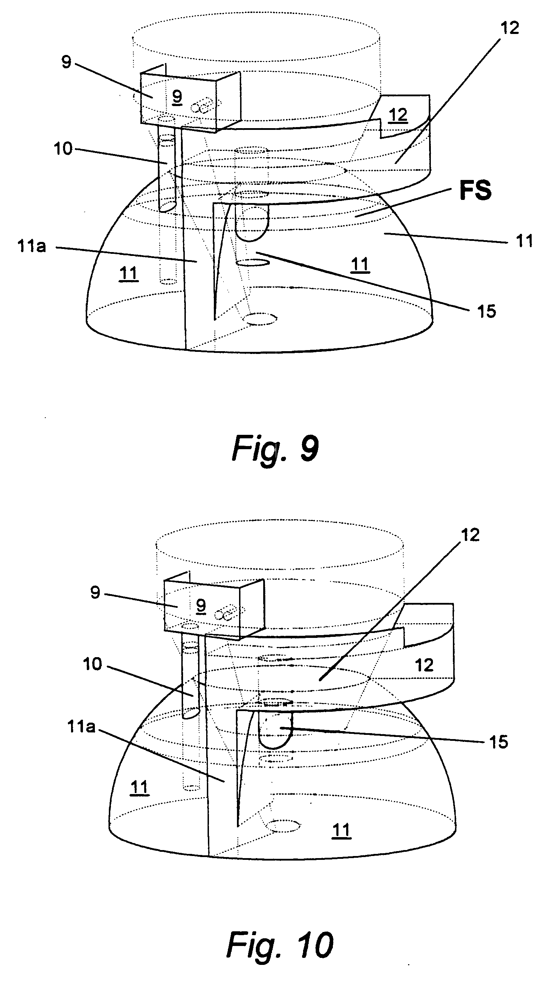

The series of Fig. 8 to Fig. 12 shows the accumulation of the biogas in the methane fermenter 11, and the press through of the floating sludge blanket (marked with FS in Fig. 8 and 9) from the methane digestor into the equalisation tank 12. This process can also easily be automated with a pressure sensor and a floater on top of the floating sludge blanket as the only sensors in the methane fermenter.

Fig. 8 shows the anaerobic unit after biogas release. Due to the high hydraulic drop between the hydrolysis device 9 and the sludge levels in the methane fermenter, this is the best moment, to release fresh hydrolysed material from the hydrolysis device 9 through pipe 10 into the methane fermenter 11 (see explanations of Fig. 7). The floating sludge blanket is accumulating at the top of the methane fermenter 11. The pressure equalisation tank 12 is empty.

Fig. 9 shows the accumulation of the biogas in the methane fermenter 11. The sludge level as well as the floating sludge blanket in the methane fermenter 11 drop, and the sludge levels in the pressure equalisation device 12 and in the floating sludge blanket removal channel 15 rise. The organic fermenting solution is pressed through connecting channel 14a from the methane fermenter 11 into the pressure equalisation tank 12. The sludge level in the pressure equalisation tank 12 has not reached the upper edge of the floating sludge blanket removal channel 15.

Fig. 10 shows the moment to release the biogas from methane fermenter 11. The sludge level in the pressure equalisation tank 12 and the floating sludge blanket removal channel 15 has reached the upper edge of the floating sludge blanket removal channel 15. The hydraulic connection from the methane fermenter 11 through channel 15 into the pressure equalisation tank 12 is open. The liquid sludge level in the methane fermenter has not yet risen so far to reach the lower edge of the floating sludge blanket removal channel 15.

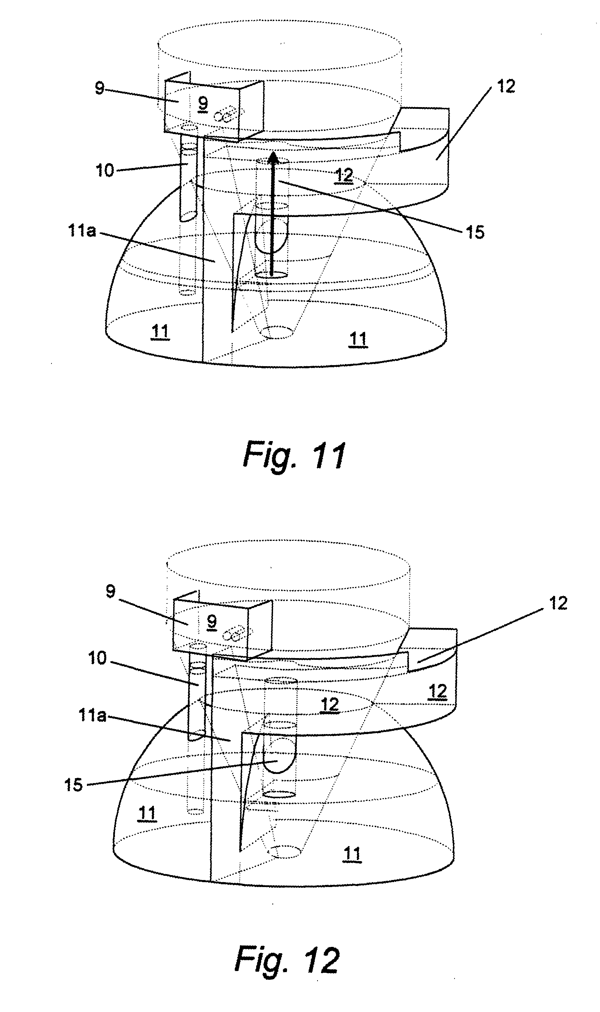

Fig. 11 shows the situation, if the biogas after the moment, described in Fig. 10, is not released. The liquid sludge level in the methane fermenter drops under the lower edge of floating sludge blanket removal channel 15. Due to the lower specific weight of the biogas bubbles containing sludge particles, these particles are ascending. During ascent, the pressure of the surrounding liquid phase decreases. The biogas bubbles in the sludge particles are thus growing, so decreasing the specific weight of the sludge particles. Therefore a suction is developing in the floating sludge blanket removal channel 15 (symbolised with the bold arrow in floating sludge blanket removal channel 15).

Fig. 12 shows the situation after the floating sludge blanket removal. The sludge blanket is floating in the pressure equalisation tank 12. Now, only gas bubbles will pass through floating sludge blanket removal channel 15. This demonstrates the double function of the floating sludge blanket removal channel 15 as an overpressure valve as well. This is the moment, to release the biogas.

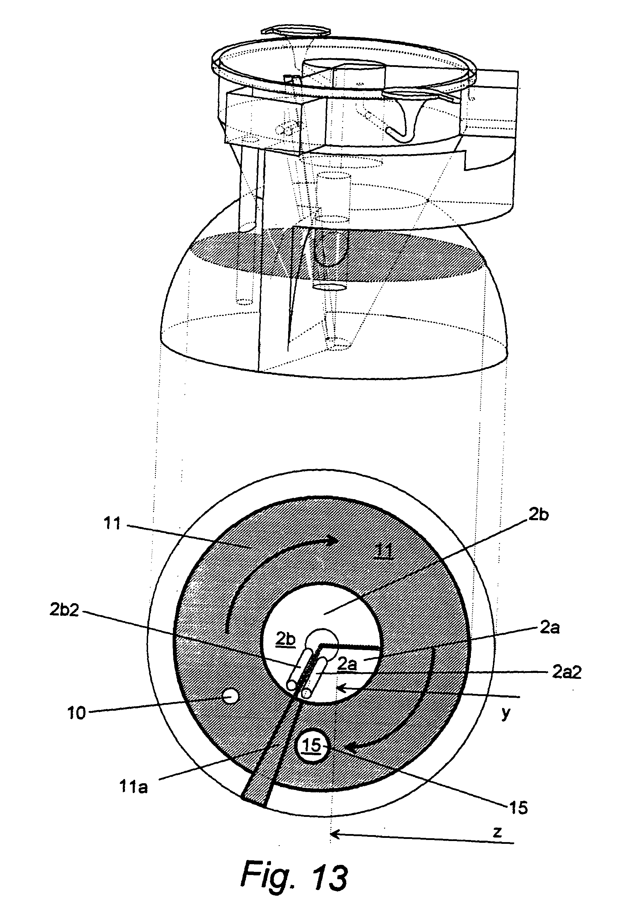

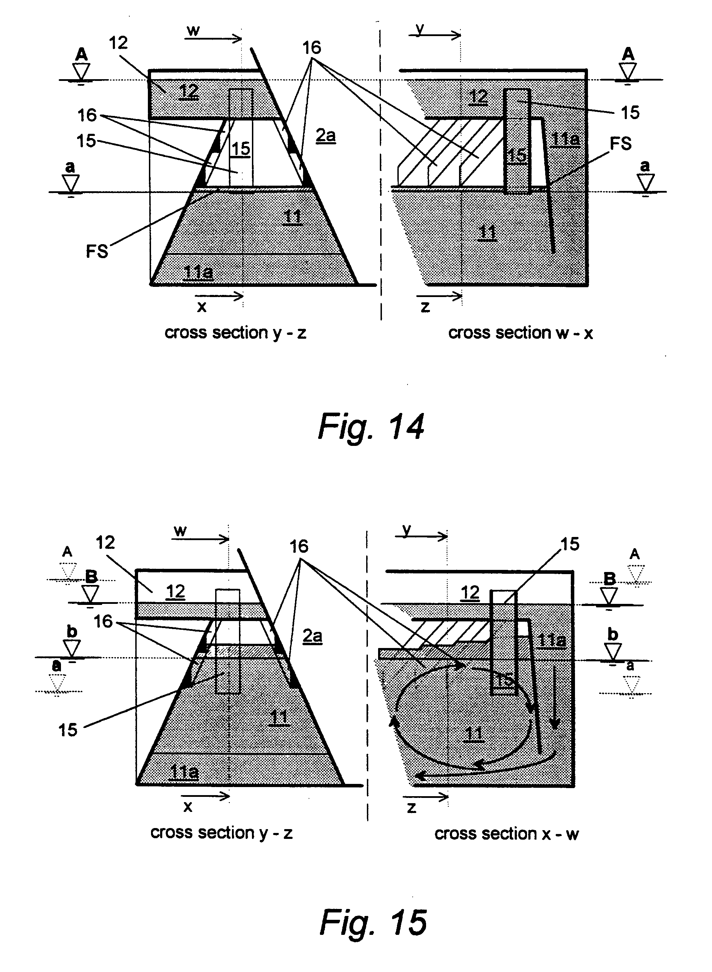

Fig. 13 shows a cross section through the methane fermenter. The hatched ellipse in the upper drawing indicates the cut level seen from top in the lower drawing. In the middle of the lower drawing the funnel top of the sedimentation devices 2a and 2b with the separation wall and the sludge outlet devices 2a2 and 2b2 are visible.
The hatched area indicates the cut through the methane fermenter 11. The material enters the methane fermenter 11 via pipe 10. It flows with a composition specific retention time around the sedimentation device through channel 14a into pressure equalisation tank. There is a back and forth flow due to the production and release of the biogas. The dotted line with the arrows and the letter y and z is indicating the location of the cross section of the following Figures 14 to 19.

The series of Fig. 14 to Fig. 19 is showing the transport mechanism of the floating sludge blanket in the methane fermenter 11 towards the floating sludge blanket removal channel 15. The same transportation system is also installed in the pressure equalisation tank 12, but not shown in the figures.
The left-hand drawings of the figures 14 to 19 are showing a radial cross section through the methane fermenter 11 and the pressure equalisation tank 12, with the view towards the floating sludge blanket removal channel 15 and the end of the methane fermenter 11. At the bottom of the left drawings, the inlet of the connecting channel 11a is visible.
The right-hand drawings of the figures 14 to 19 are showing a tangential cross section through the methane fermenter 11, the connecting channel 11a, the floating sludge blanket removal channel 15 and the equalisation tank 12 with the view towards the outer wall of the methane fermenter.
The floating sludge blanket transporting bars 16 are fixed at the inner and outer walls of the methane fermenter. As visible, the lower edge of the sludge blanket transporting bars 16 is horizontal increasing and the inner edge is vertically increasing.

If the sludge blanket is rising, the lower edges of the transportation bars 16 cut the sludge blanket and moves it during its rising towards floating sludge blanket removal channel 15. At the same time, the floating sludge blanket is compressed by the walls of the methane fermenter 11.
If the sludge blanket drops, the transportation bars 16 and the walls of the methane fermenter 11 release the floating sludge blanket. It is sliding downwards at the vertical edges of the transportation bars 16. The fibrous structure of the sludge blanket prevents a decompression of the sludge blanket. The slow drop of the sludge level due to the biogas production press it slightly towards the floating sludge blanket removal channel 15. The sludge blanket is also fixed during level drop due to the fact that the floating sludge blanket removal channel 15 sticks in the sludge blanket.
The floating sludge blanket is drawn hatched, the liquid sludge is underlaied with black points. At the outer left and right side of each drawing, the liquid sludge levels are marked with triangles. Capital bold letters are indicating the levels in the pressure equalisation tank 12, small bold letters the levels in the methane fermenter 11. Broken triangular level marks with normal letters are indicating levels of the former drawings as to demonstrate the level differences.

Fig. 14 shows the situation before biogas release (see also Fig. 10 and explanations). The floating sludge blanket is equally spread over the upper liquid sludge surface.

Fig. 15 shows the situation during biogas release. The fast back flow of liquid sludge from the pressure equalisation tank 12 through the lower opening of the connection channel 11a into the methane fermenter 11 induces a convection roll, marked with the arrows in the drawing, supporting additionally the movement of the floating sludge blanket towards the floating sludge blanket removal channel 15.

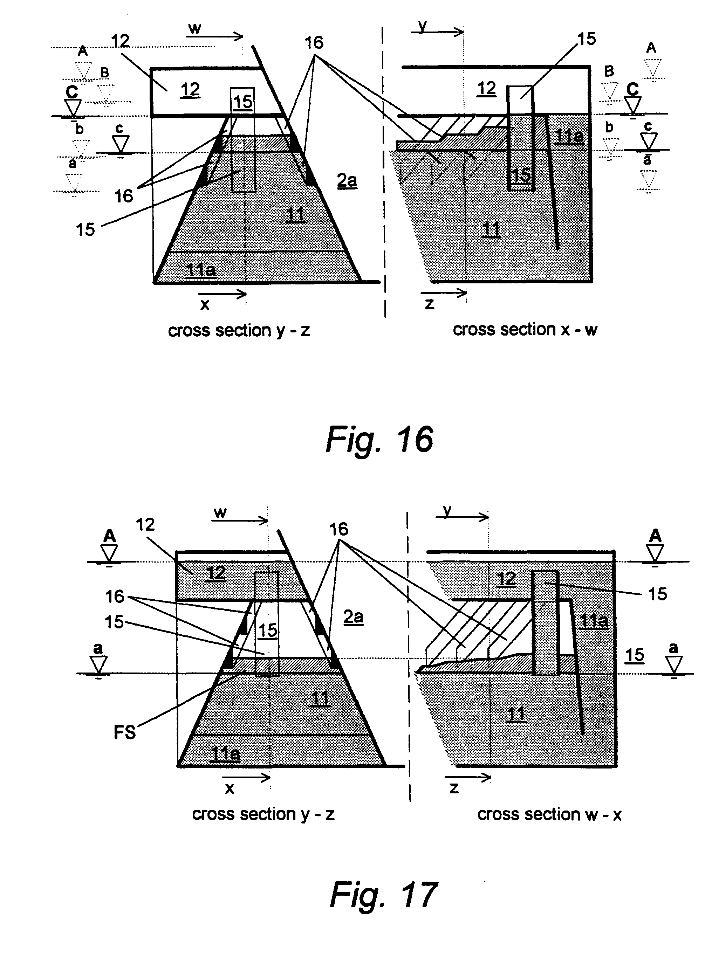

Fig. 16 shows the compressed sludge blanket after biogas release (see also Fig. 7 and explanations). Now the sludge level in the methane fermenter 11 starts to slowly drop due to biogas accumulation at the top of the methane fermenter 11.

Fig. 17 shows the situation before normally biogas release (see also Fig. 10 and explanations).

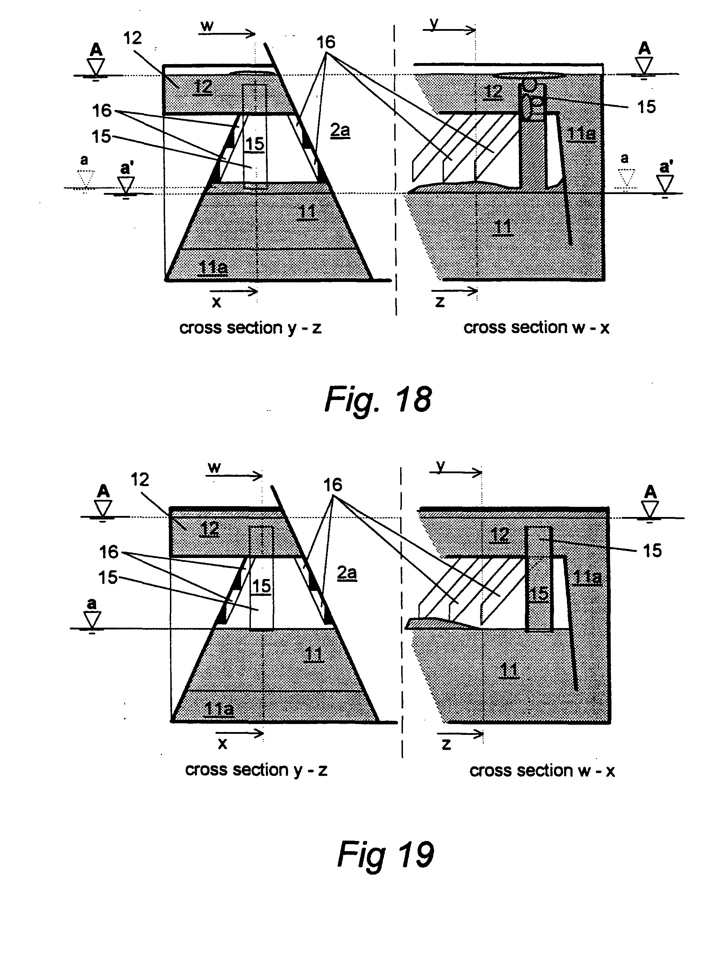

Fig. 18 shows the floating sludge blanket removal from the methane fermenter 11 into the pressure equalisation tank 12 (see also Fig. 11 and explanations).

Fig. 19 shows the removed floating sludge blanket. It is now floating in the pressure equalisation tank 12, in which it is transported with the same transportation bars and changing sludge levels towards the outlet to overflow into the drainage and/or drying device 13.

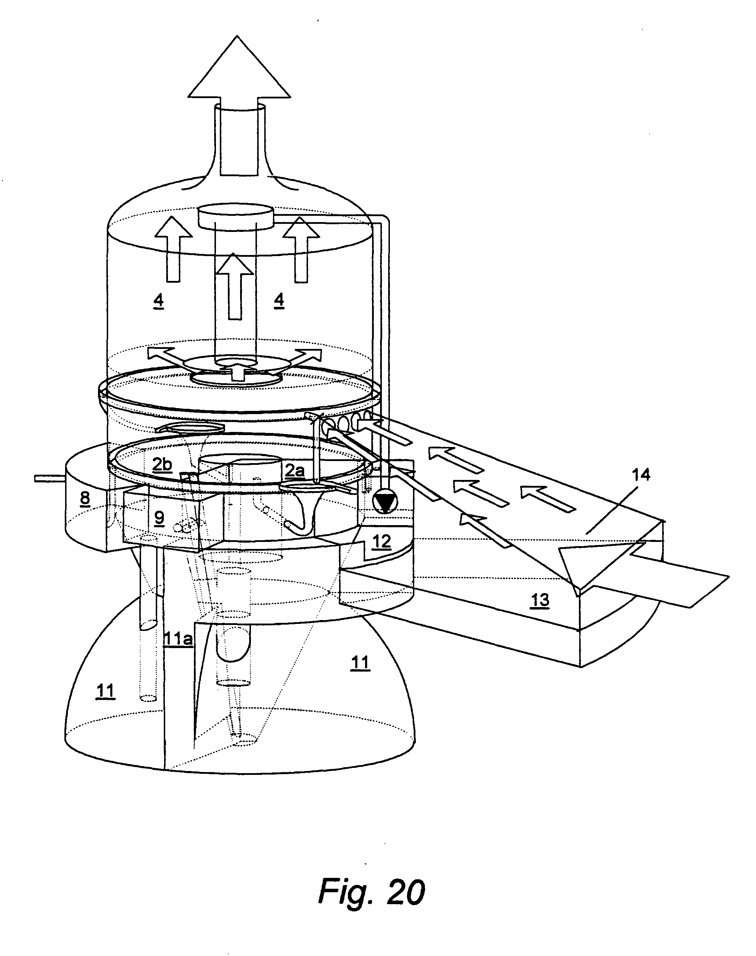

Fig 20 shows the air flow caused by the strengthened chimney effect within the device according to the invention. The air is entering between the draining and/or drying device 13 and the preferably transparent cover 14. After heating up with solar thermal energy strengthened by the greenhouse effect of the covered area and moving due to the slope of the cover towards the air inlet 14a, the air is then entering the tower between the sedimentation device 2a and 2b and the primary treatment device 4. With an appropriate air stream device it can be used here to transport the floating sludge blankets of the sedimentation device 2a and 2b towards a blow off device (not shown). From here, it flows through the air inlet 4a to aerate the primary treatment device 4. It leaves the device according to the invention through the chimney on top of the primary treatment device.

### List of devices:

- device 1:: Inlet building with sand trap
- device 1a:: Inlet pipe between inlet building 1 and inlet cylinder 2a1
- device 2a:: Exemplary sedimentation device 1 (primary sludge)
- device 2a1:: Inlet cylinder of exemplary sedimentation device 1 2a
- device 2a2:: Sludge blow off pipe of exemplary sedimentation device 1 2a
- device 2a3:: Connection pipe between sludge blow off pipe 2a2 and hydrolysis and/or mixing tank 9 with valve.
- device 2b:: Exemplary sedimentation device 2 (secondary bacterial sludge)
- device 2b1:: Inlet cylinder of exemplary sedimentation device 2 2b
- device 2b2:: Sludge blow off pipe of exemplary sedimentation device 2 2b
- device 2b3:: Connection pipe between sludge blow off pipe 2b2 and hydrolysis and/or mixing tank 9 with valve.
- device 3:: Pump sump for distributor 4b
- device 3a:: Pipe between pump sump 3 and distributor 4b
- device 4:: Primary treatment device for waste water (purification up to full nitrification)
- device 4a:: Air inlet device for device for waste water purification 4
- device 4b:: Waste water distributor of device for waste water purification 4
- device 5:: Gutter of device from primary treatment 4
- device 5a:: Connection pipe with valve between gutter 5 and pump sump 3
- device 5b:: Connection pipe with valve between gutter 5 and inlet building 1
- device 5c:: Connection pipe with valve between gutter 5 and inlet building 6
- device 6:: Inlet building for exemplary sedimentation device 2 2b
- device 6a:: Inlet pipe between inlet building 6 and inlet cylinder 2b1
- device 7:: Gutters of sedimentation devices (2a and 2b)
- device 7a:: Gutter of sedimentation device 1 2a
- device 7b:: Gutter of sedimentation device 2 2b
- device 7c:: Connection pipe with valve between gutter 7b and secondary treatment 8
- device 7d:: Connection pipe with valve between gutter 7a and pump sump 3 for primary treatment 8
- device 7e:: Connection pipe with valve between gutter 7b and pump sump 3 for primary treatment 8
- device 8:: Secondary treatment (Denitrification and Phosphorus retaining device)
- device 8a:: Outlet pipe for disposal or further treatment
- device 9:: Hydrolysis and/or mixing tank.
- device 10:: Connection pipe between hydrolysis and/or mixing tank 9 and methane fermenter 11
- device 11:: Methane fermenter
- device 11a:: Connection channel between methane fermenter 11 and pressure equalisation tank 12
- device 12:: pressure equalisation tank
- device 13:: Drainage and/or drying beds
- device 14:: Covering device for drainage or drying beds 13
- device 14a:: Air inlet
- device 15:: Floating sludge blanket removal channel
- device 16:: Floating sludge blanket transporting bars in the methane fermenter 11
- device 17:: Composting device

## Claims

1. A method for the combined treatment of sewage and/or waste water, and/or sludges, said siudges being obtainable by treatment of sewage and/or waste water, and size reduced biodegradable domestic organic garbage, wherein said sewage and/or waste water together with said garbage, after a usual mechanical pre-treatment including fat and grease removal, is processed by the following steps:
(a) a first waste water and/or sewage liquid - solid separation;
(b) a primary biological sewage and/or waste water treatment step of the liquid of step (a);
(c) a second liquid - solid separation of the treated product of step (b);
(d) an anaerobic hydrolytic treatment of said sludge produced in step (a) and/or total or parts of sludge produced in step (c) and said garbage, wherein said anaerobic treatment is stationarily hydraulically directly or indirectly connected to (a), (c) and/or (e); and
(e) anaerobic methane fermentation treatment of said sludge produced in step (a) and/or total or parts of said sludge produced in step (c) and said garbage from which the wooden compounds have been essentially removed together with the fats and grease that were presently removed from said water, wherein said anaerobic treatment is stationarily hydraulically directly or indirectly connected to (d).

2. Method according to claim 1, **characterised by** a secondary biological, anaerobic waste water treatment between steps (c) and (d).

3. Method according to claim 1 or 2, **characterised by** a third waste water liquid-solid separation between steps (c) and (d) and/or between steps (d) and (e) and/or after step (e).

4. Method according to claim 3, **characterised in that** the third liquid - solid separation is a sedimentation.

5. Method according to claim 4, **characterised by** an anaerobic draining and/or drying treatment of fermented matter obtained by step (d) and (e).

6. Method according to claim 5, **characterised in that** the aerobic draining and/or drying treatment is carried out preferably together with the wooden compounds that were previously removed from said garbage.

7. Method according to any of the preceding claims, **characterised by** an aerobic composting treatment of the fermented and dry matter obtained in step (e).

8. Method according to claim 7, **characterised in that** the composting treatment is carried out together with the wooden components that were previously removed from said garbage.

9. Method of any of the preceding claims, **characterised by** the step of providing a liquid flow caused by a chimney effect for enhancing the biological activity of the primary waste water treatment device; and/or for minimising the development of offensive odours of the first and/or the second liquid - solid separation, preferably sedimentation and/or the drainage and/or drying devices; and/or for transportation of the floating sludges of the primary and/or secondary liquid - solid separation, preferably sedimentation and/or a waste water treatment device.

10. Method according to any of the preceding claims, **characterised by** the steps of moving and/or removing the floating sludge blankets in/from an anaerobic reactor by applying gas pressure exerted by the biogas, said biogas being produced by said sludges and/or said garbage.

11. Method according to claim 9 or 10, **characterised by** enhancing the chimney effect by directing air warmed up by the greenhouse effect generated preferably in the draining and/or drying device into said liquid flow.

12. Device for carrying out a method according to any of the preceding claims comprising:
(a) means adapted to carry out a first waste water and/or sewage liquid - solid separation;
(b) means being adapted to carry out a primary biological sewage and/or waste water treatment step of the liquid obtained in the liquid - solid separation;
(c) means adapted for a second liquid - solid separation of the obtained product;
(d) means adapted for an anaerobic hydrolytic treatment of said sludge, wherein the hydrolytic treatment means is stationarily hydraulically directly or indirectly connected to the first sewage liquid - solid separation means, the second liquid - solid separation means and/or a means adapted for an anaerobic methane fermentation treatment;
(e) the anaerobic methane fermentation treatment means which is adopted to treat said sludge from the first sewage liquid - solid separation means and/or total or parts of said sludge produced by the second liquid - solid separation means and said garbage from which the wooden compounds have been essentially removed wherein the anaerobic methane fermentation treatment means is stationarily hydraulically directly or indirectly connected to the anaerobic hydrolytic treatment means.

13. Device according to claim 12, **characterised by** a fixed dome plant preferably with a funnel as bottom for avoidance of accumulation of solids by their release by water pressure.

14. Device according to claim 12 or 13, **characterised by** being built around, and/or under a liquid - solid separation, preferably sedimentation device, but being hydraulically separated by at least one inbetween arranged tank.

15. Device according to any one of claims 12 to 14, **characterised in that** the liquid - solid separation, preferably sedimentation device is a Dortmund Tank.

16. Device according to claim 15, **characterised by** means for sedimentating primary sewage sludge and secondary bacterial sludge in one Dortmund Tank, divided vertically by a wall.

17. Device according to any one of claims 12 to 16, **characterised by** the primary waste water treatment device being arranged upon the liquid - solid separation, preferably sedimentation device.

18. Device according to any one of claims 12 to 17, **characterised by** building the combination in form of a closed tower with preferably only one air inlet.

19. Device according to any one of claims 12 to 18, **characterised by** combined denitrification and phosphorus retaining with an anaerobic fixed bed or soil filter, made out of Goethite, Hematite, or other minerals with iron-active surfaces.

20. Device according to any one of claims 12 to 19, by comprising an area covered with a preferably transparent cover with a slope upwards towards the direction of the intended air flow.

21. Device according to any of claims 12 to 20, **characterised by** being connected with at least one foreign energy consuming device.

22. Device according to any of claims 12 to 20, **characterised by** comprising a drainage and/or drying device and/or a composting device.

23. Device according to any of claims 12 to 20, **characterised by** utilizing 0.05 to 0.5 m² per PE.

## Patentansprüche

1. Verfahren für die kombinierte Behandlung von Schmutzwasser oder Abwasser, und/oder Schlämmen, wobei die Schlämme durch die Behandlung von Schmutzwasser und/oder Abwasser erhalten werden, und von größenreduzierten biologisch abbaubaren organischen Hausmüll, wobei das Schmutzwasser und/oder Abwasser und/oder der Müll nach einer eine Entfernung von Fett und Schmiere einschließenden üblichen mechanischen Vorbehandlung mittels der nachstehenden Schritte verarbeitet werden:
(a) einer ersten Flüssigkeit/Feststoff-Trennung des Abwassers und/oder Schmutzwassers;
(b) einem primären biologischen Behandlungsschritt des Schmutzwassers und/oder Abwassers der Flüssigkeit von Schritt (a);
(c) einer zweiten Flüssigkeit/Feststoff-Trennung des behandelten Produkts von Schritt (b);
(d) einer anaeroben Hydrolysebehandlung des in Schritt (a) erzeugten Schlamms und/oder des gesamten oder von Teilen des in Schritt (c) erzeugten Schlamms und des Mülls, wobei die anaerobe Trennung stationär hydraulisch direkt oder indirekt mit (a), (c) und/oder (e) verbunden ist; und
(e) einer anaeroben Methanfermentationsbehandlung des in Schritt (a) erzeugten Schlamms und/oder des gesamten oder von Teilen des in Schritt (c) erzeugten Schlamms und des Mülls, aus welchem die Holzbestandteile im wesentlichen zusammen mit den Fetten und der Schmiere entfernt wurden, welche derzeit aus dem Wasser entfernt wurden, wobei die anaerobe Behandlung stationär hydraulisch direkt oder indirekt mit (d) verbunden ist.

2. Verfahren nach Anspruch 1, **gekennzeichnet durch** eine sekundäre biologische anaerobe Abwasserbehandlung zwischen den Schritten (c) und (d).

3. Verfahren nach Anspruch 1 oder 2, **gekennzeichnet durch** eine dritte Flüssigkeit/Feststoff-Trennung des Abwassers zwischen den Schritten (c) und (d) und/oder zwischen den Schritten (d) und (e) und/oder nach dem Schritt (e).

4. Verfahren nach Anspruch 3, **dadurch gekennzeichnet, daß** die dritte Flüssigkeit/Feststoff-Trennung eine Sedimentation ist.

5. Verfahren nach Anspruch 4, **gekennzeichnet durch** eine anaerobe Entwässerungs- und/oder Trocknungsbehandlung der **durch** die Schritte (d) und (e) erhaltenen fermentierten Masse.

6. Verfahren nach Anspruch 5, **dadurch gekennzeichnet, daß** die aerobe Entwässerungs- und/oder Trocknungsbehandlung bevorzugt mit den Holzbestandteilen durchgeführt wird, welche zuvor aus dem Müll entfernt wurden.

7. Verfahren nach einem der vorstehenden Ansprüche, **gekennzeichnet durch** eine aerobe Kompostierungsbehandlung der in Schritt (e) erhaltenen fermentierten und trockenen Masse.

8. Verfahren nach Anspruch 7, **dadurch gekennzeichnet, daß** die Kompostierungsbehandlung zusammen mit den Holzbestandteilen ausgeführt wird, welche zuvor aus dem Müll entfernt wurden.

9. Verfahren nach einem der vorstehenden Ansprüche, **gekennzeichnet durch** den Schritt der Erzeugung einer **durch** einen Kamineffekt bewirkten Flüssigkeitsströmung zur Verbesserung der biologischen Aktivität der primären Abwasserbehandlungsvorrichtung; und/oder zur Minimierung der Entwicklung störender Gerüche der ersten und/oder zweiten Flüssigkeit/Feststoff-Trennungs-, bevorzugt Sedimentations- und/oder Entwässerungs- und/oder Trocknungsvorrichtungen; und/oder für den Transport der aufschwimmenden Schlämme der primären und/oder sekundären Flüssigkeit/Feststoff-Trennungs-, bevorzugt einer Sedimentations- und/oder Abwasserbehandlungsvorrichtung.

10. Verfahren nach einem der vorstehenden Ansprüche, **gekennzeichnet durch** die Schritte der Verschiebung und/oder Entfernung der aufschwimmenden Schlammdecken in/aus einem anaeroben Reaktor **durch** Aufbringen eines **durch** das Biogas ausgeübten Gasdrucks, wobei das Biogas **durch** die Schlämme und/oder den Müll erzeugt wird.

11. Verfahren nach Anspruch 9 oder 10, **gekennzeichnet durch** die Verbesserung des Kamineffekts **durch** Einleiten **durch** den Treibhauseffekt erzeugter warmer Luft in den Flüssigkeitsstrom bevorzugt in der Entwässerungs- und/oder Trocknungsvorrichtung.

12. Vorrichtung zum Ausführen eines Verfahrens gemäß einem der vorstehenden Ansprüche, mit:
(a) einer Einrichtung, die zum Ausführen einer ersten Flüssigkeit/Feststoff-Trennung von Schmutzwasser und/ oder Abwasser angepaßt ist;
(b) einer Einrichtung, die zum Ausführen eines primären biologischen Behandlungsschrittes von Schmutzwasser und/oder Abwasser in der Flüssigkeit/Feststoff-Trennung erhaltenen Flüssigkeit angepaßt ist;
(c) einer Einrichtung, die für eine zweite Flüssigkeit/Feststoff-Trennung des erhaltenen Produkts angepaßt ist;
(d) einer Einrichtung, die für eine anaerobe Hydrolysebehandlung des Schlamms angepaßt ist, wobei die Hydrolysebehandlungseinrichtung stationär hydraulisch direkt oder indirekt mit der ersten Abwasser/Flüssigkeits-Trenneinrichtung, der zweiten Flüssigkeit/ Feststoff-Trenneinrichtung und/oder einer für eine anaerobe Fermentationsbehandlung angepaßten Einrichtung verbunden ist; und
(e) der anaeroben Methanfermentationsbehandlungseinrichtung, welche für die Behandlung des Schlamms aus der ersten Flüssigkeit/Feststoff-Trenneinrichtung des Abwasser und/oder des gesamten oder von Teilen des durch die zweite Flüssigkeit/Feststoff-Trenneinrichtung erzeugten Schlamms und des Mülls, von welchem die Holzbestandteile im wesentlichen getrennt wurden, angepaßt ist, wobei die anaerobe Methanfermentationseinrichtung stationär hydraulisch direkt oder indirekt mit der anaeroben Hydrolysebehandlungseinrichtung verbunden ist.

13. Vorrichtung nach Anspruch 12, **gekennzeichnet durch** eine feste Domanlage, bevorzugt mit einem Trichter als Boden, zur Vermeidung der Ansammlung von Feststoffen aufgrund deren Lösung **durch** den Wasserdruck.

14. Vorrichtung nach Anspruch 12 oder 13, **dadurch gekennzeichnet, daß** sie um und/oder unter einer Flüssigkeit/ Feststoff-Trennungs-, bevorzugt Sedimentationsvorrichtung gebaut ist, aber hydraulisch durch mindestens einen dazwischen angeordneten Tank getrennt ist.

15. Vorrichtung nach einem der Ansprüche 12 bis 14, **dadurch gekennzeichnet, daß** die Flüssigkeit/Feststoff-Trennungs-, bevorzugt Sedimentationsvorrichtung ein Dortmundbrunnen ist.

16. Vorrichtung nach Anspruch 15, **gekennzeichnet durch** eine Einrichtung zur Sedimentation des primären Abwasserschlamms und des sekundären Bakterienschlamms in einem **durch** eine vertikale Wand unterteilten Dortmundbrunnen.

17. Vorrichtung nach einem der Ansprüche 12 bis 16, **dadurch gekennzeichnet, daß** die primäre Abwasservorrichtung auf der Flüssigkeit/Feststoff-Trennungs-, bevorzugt über der Sedimentationsvorrichtung angeordnet ist.

18. Vorrichtung nach einem der Ansprüche 12 bis 17, **dadurch gekennzeichnet, daß** die Kombination in der Form eines geschlossenen Turms bevorzugt mit nur einem Lufteinlaß gebaut wird.

19. Vorrichtung nach einem der Ansprüche 12 bis 18, **gekennzeichnet durch** eine kombinierte Denitrifikation und Phosphorzurückhaltung mit einem anaeroben Festbett oder Bodenfilter bestehend aus Goethit und Hämatit oder anderen Mineralien mit eisenaktiven Oberflächen.

20. Vorrichtung nach einem der Ansprüche 12 bis 19, **dadurch gekennzeichnet, daß** sie einen Bereich aufweist, der bevorzugt mit einem transparenten Deckel, mit einer Aufwärtsneigung in der Richtung des gewollten Luftstroms abgedeckt ist.

21. Vorrichtung nach einem der Ansprüche 12 bis 20, **dadurch gekennzeichnet, daß** sie mit mindestens einer fremden Energieverbrauchsvorrichtung verbunden ist.

22. Vorrichtung nach einem der Ansprüche 12 bis 20, **dadurch gekennzeichnet, daß** sie eine Entwässerungs- und/oder Trocknungs- und/oder Kompostierungsvorrichtung aufweist.

23. Vorrichtung nach einem der Ansprüche 12 bis 20, **gekennzeichnet durch** eine Nutzung von 0,05 bis 0,5 m² pro PE.

## Revendications

1. Procédé pour le traitement combiné d'eaux d'égout et/ou d'eaux usées et/ou de boues, lesdites boues pouvant être obtenues par traitement d'eaux d'égout et/ou d'eaux usées, et de déchets organiques domestiques biodégradables de taille réduite, dans lequel lesdites eaux d'égout et/ou eaux usées ainsi que lesdits déchets, après un prétraitement mécanique habituel incluant la séparation des corps gras et de la graisse, sont traités par les étapes suivantes :
(a) une première séparation liquide-solide des eaux usées et/ou des eaux d'égout ;
(b) une étape de traitement biologique primaire des eaux d'égout et/ou eaux usées du liquide de l'étape (a) ;
(c) une seconde séparation liquide-solide du produit traité de l'étape (b) ;
(d) un traitement hydrolytique anaérobie de ladite boue produite dans l'étape (a) et/ou de la totalité ou de parties de la boue produite dans l'étape (c) et desdits déchets, dans lequel ledit traitement anaérobie est stationnairement hydrauliquement directement ou indirectement relié à (a), (c) et/ou (e) ; et
(e) un traitement de fermentation anaérobie méthanique de ladite boue produite dans l'étape (a) et/ou de la totalité ou de parties de ladite boue produite dans l'étape (c) et desdits déchets dont les composés du bois ont été essentiellement séparés avec les corps gras et la graisse qui étaient déjà séparés de ladite eau, ledit traitement anaérobie étant stationnairement hydrauliquement directement ou indirectement relié à (d).

2. Procédé selon la revendication 1, **caractérisé par** un traitement biologique anaérobie secondaire des eaux usées entre les étapes (c) et (d).

3. Procédé selon la revendication 1 ou 2, **caractérisé par** une troisième séparation liquide-solide des eaux usées entre les étapes (c) et (d) et/ou entre les étapes (d) et (e) et/ou après l'étape (e).

4. Procédé selon la revendication 3, **caractérisé en ce que** la troisième séparation liquide-solide est une sédimentation.

5. Procédé selon la revendication 4, **caractérisé par** un traitement anaérobie de drainage et/ou séchage de la matière fermentée obtenue par les étapes (d) et (e).

6. Procédé selon la revendication 5, **caractérisé en ce que** le traitement aérobie de drainage et/ou séchage est mis en oeuvre de préférence avec les composés de bois qui ont été préalablement séparés des déchets.

7. Procédé selon l'une quelconque des revendications précédentes, **caractérisé par** un traitement de compostage aérobie de la matière fermentée et sèche obtenue dans l'étape (e).

8. Procédé selon la revendication 7, **caractérisé en ce que** le traitement de compostage est effectué avec les constituants de bois qui ont été préalablement séparés desdits déchets.

9. Procédé selon l'une quelconque des revendications précédentes, **caractérisé par** l'étape consistant à fournir un écoulement liquide provoqué par un effet cheminée pour accroître l'activité biologique du dispositif de traitement primaire des eaux usées ; et/ou pour minimiser le développement de mauvaises odeurs des dispositifs de première et/ou seconde séparation liquide-solide, de préférence de sédimentation, et/ou de drainage et/ou de séchage ; et/ou pour transporter les boues flottantes du dispositif de séparation liquide-solide primaire et/ou secondaire, de préférence de sédimentation, et/ou de traitement des eaux usées.

10. Procédé selon l'une quelconque des revendications précédentes, **caractérisé par** les étapes consistant à déplacer et/ou séparer les lits de boue flottante dans/depuis un réacteur anaérobie en appliquant une pression de gaz exercée par le biogaz, ledit biogaz étant produit par lesdites boues et/ou lesdits déchets.

11. Procédé selon la revendication 9 ou 10, **caractérisé par** l'accroissement de l'effet cheminée en envoyant un air chauffé par l'effet de serre généré de préférence dans le dispositif de drainage et/ou séchage jusque dans ledit écoulement liquide.

12. Dispositif pour mettre en oeuvre un procédé selon l'une quelconque des revendications précédentes, comprenant :
(a) des moyens adaptés pour effectuer une première séparation liquide-solide d'eaux usées et/ou d'eaux d'égout ;
(b) des moyens qui sont adaptés pour effectuer une étape de traitement biologique primaire d'eaux d'égout et/ou d'eaux usées du liquide obtenu dans la séparation liquide-solide ;
(c) des moyens adaptés pour une seconde séparation liquide-solide du produit obtenu ;
(d) des moyens adaptés pour un traitement hydrolytique anaérobie de ladite boue, dans lesquels le moyen de traitement hydrolytique est stationnairement hydrauliquement directement ou indirectement relié aux moyens de première séparation liquide-solide, aux moyens de seconde séparation liquide-solide et/ou à un moyen adapté pour un traitement de fermentation anaérobie méthanique ;
(e) le moyen de traitement de fermentation anaérobie méthanique qui est adapté pour traiter ladite boue des moyens de première séparation liquide-solide d'eaux d'égout et/ou la totalité ou des parties de ladite boue produite par les moyens de seconde séparation liquide-solide et lesdits déchets dont les composés de bois ont été essentiellement éliminés, le moyen de traitement de fermentation anaérobie méthanique étant stationnairement hydrauliquement directement ou indirectement relié au moyen de traitement hydrolytique anaérobie.

13. Dispositif selon la revendication 12, **caractérisé par** une installation à dôme fixe de préférence avec un entonnoir en tant que fond pour éviter l'accumulation des matières solides en les évacuant par la pression d'eau.

14. Dispositif selon la revendication 12 ou 13, **caractérisé en ce qu'**il est construit autour, et/ou en dessous d'un dispositif de séparation liquide-solide, de préférence de sédimentation, mais est séparé hydrauliquement par au moins un réservoir intercalé.

15. Dispositif selon l'une quelconque des revendications 12 à 14, **caractérisé en ce que** le dispositif de séparation liquide-solide, de préférence de sédimentation, est une fosse Dortmund.

16. Dispositif selon la revendication 15, **caractérisé par** des moyens pour sédimenter une boue d'égout primaire et une boue bactérienne secondaire dans une fosse Dortmund, divisée verticalement par une paroi.

17. Dispositif selon l'une quelconque des revendications 12 à 16, **caractérisé en ce que** le dispositif de traitement primaire des eaux usées est disposé sur le dispositif de séparation liquide-solide, de préférence de sédimentation.

18. Dispositif selon l'une quelconque des revendications 12 à 17, **caractérisé par** la construction de la combinaison sous la forme d'une tour fermée avec de préférence seulement une entrée d'air.

19. Dispositif selon l'une quelconque des revendications 12 à 18, **caractérisé par** la dénitrification et la rétention du phosphore combinées avec un lit fixe anaérobie ou un filtre à salissure, fait en goethite, hématite ou d'autres minéraux ayant des surface de fer actives.

20. Dispositif selon l'une quelconque des revendications 12 à 19, **caractérisé en ce qu'**il comprend une zone recouverte avec un couvercle de préférence transparent avec une pente remontant vers la direction du flux d'air prévu.

21. Dispositif selon l'une quelconque des revendications 12 à 20, **caractérisé en ce qu'**il est relié avec au moins un dispositif étranger consommateur d'énergie.

22. Dispositif selon l'une quelconque des revendications 12 à 20, **caractérisé en ce qu'**il comprend un dispositif de drainage et/ou séchage et/ou un dispositif de compostage.

23. Dispositif selon l'une quelconque des revendications 12 à 20, **caractérisé en ce qu'**il utilise de 0,05 à 0,5 m² par PE.
